# EUROPEAN PATENT SPECIFICATION

(11) **EP 4 512 376 B1**
(45) Date of publication and mention of the grant of the patent: **21.10.2026**
(21) Application number: 24221729.7
(22) Date of filing: 18.02.2020
(51) Int. Cl.: F16L 55/163, A61F 2/82, A61F 2/90, F16L 55/18, F16L 57/02, A61F 2/915

(54) **STENT SPRINGS AND STENTS FOR REPAIRING PIPES**
STENTFEDERN UND STENTS ZUR REPARATUR VON ROHREN
RESSORTS ET STENTS POUR LA RÉPARATION DE TUYAUX

(30) Priority: 19.02.2019 US 201962807264 P; 15.04.2019 US 201962834168 P
(43) Date of publication of application: 26.02.2025
(62) Document number(s) of the earlier application(s) in accordance with Art. 76 EPC: 20758706.4 / 3 928 022
(73) Proprietor: Mueller International, LLC, Atlanta, GA 30328 (US)
(72) Inventor: FURCOIU, Aurelian loan, Chattanooga 37412 (US)
(74) Representative: Page White Farrer

(56) References cited:
- KR-A- 20070 018 627
- US-A- 5 645 559
- US-A1- 2009 248 132
- US-A1- 2012 259 404

## Description

### TECHNICAL FIELD

This disclosure relates to the field of pipe repair. More specifically, this disclosure relates to stent springs and stents for repairing a pipe.

### BACKGROUND

Piping systems, including municipal water systems, can develop breaks in pipe walls that can cause leaking. Example of breaks in a pipe wall can include radial cracks, axial cracks, point cracks, etc. Repairing a break in a pipe wall often requires the piping system to be shut off, which can be inconvenient for customers and costly for providers. Further, repairs can necessitate grandiose construction, including the digging up of streets, sidewalks, and the like, which can be costly and time-consuming.

US5645559A discloses a radially self-expanding stent having multiple layers includes a medial region and proximal and distal cuffs having diameters greater than the medial region diameter when the stent is in the relaxed state. A silicone coating circumscribes at least the medial region of the stent. A deployment device for the stent includes an inner tube surrounded by the stent along with an outer tube that radially compresses the stent.

US2012/259404A1 discloses a stent having a generally cylindrical body formed from a single woven nitinol wire. The distal and proximal ends of the stent include a plurality of loops, some of which include marker members used for visualizing the position of the stent.

US2009/248132A1 discloses a double walled stent system particularly suited for treating abnormalities of the right ventricular outflow tract is disclosed having an exterior stent component and an interior stent component. The exterior stent component includes a plurality of longitudinally-extending connectors such as straight or sinusoidal bands.

KR2007/0018627A discloses a mesh wire mesh installation method for repairing a non-excavated pipeline, in which a reinforcing mesh wire mesh is manufactured in a small-diameter form in a small diameter pipeline repair site, which is difficult for an operator to enter in performing a pipeline repair work.

### SUMMARY

The invention is defined by the appended independent claims. Particular embodiments are set out in the dependent claims.

### BRIEF DESCRIPTION OF THE DRAWINGS

The features and components of the following figures are illustrated to emphasize the general principles of the present disclosure. Corresponding features and components throughout the figures may be designated by matching reference characters for the sake of consistency and clarity.
FIG. 1A is a top perspective view of a stent, in accordance with an example useful for understanding the present disclosure, comprising a stent spring and a seal.
FIG. 1B is a top perspective view of the stent spring of FIG. 1A.
FIG. 2 is a top perspective view of the stent spring, in accordance with another example useful for understanding the present disclosure.
FIG. 3 is a top perspective view of the stent spring, in accordance with another example useful for understanding the present disclosure.
FIG. 4A is a perspective view of the stent spring, in accordance with another example useful for understanding the present disclosure, wherein the stent spring is in a rolled configuration.
FIG. 4B is perspective view of the stent spring of FIG. 4A, wherein the stent spring is in an unrolled configuration.
FIG. 5A is a bottom perspective view of the stent spring, in accordance with another example useful for understanding the present disclosure, in the rolled configuration.
FIG. 5B is perspective view of the stent spring of FIG. 5A in the unrolled configuration.
FIG. 6A is a top perspective view of the stent spring, in accordance with another example useful for understanding the present disclosure, in the rolled configuration.
FIG. 6B is perspective view of the stent spring of FIG. 6A in the unrolled configuration.
FIG. 7A is a top perspective view of the stent spring, in accordance with another example useful for understanding the present disclosure, in the rolled configuration.
FIG. 7B is perspective view of the stent spring of FIG. 7A in the unrolled configuration.
FIG. 8A is a top perspective view of the stent spring, in accordance with another example useful for understanding the present disclosure, in the rolled configuration.
FIG. 8B is front view of the stent spring of FIG. 8A in the unrolled configuration.
FIG. 9A is a top perspective view of the stent spring, in accordance with another example useful for understanding the present disclosure, in the rolled configuration.
FIG. 9B is perspective view of the stent spring of FIG. 9A in the unrolled configuration.
FIG. 10 is a top perspective view of the stent spring, in accordance with another example useful for understanding the present disclosure.
FIG. 11 is a top perspective view of the stent spring, according to an aspect of the present disclosure.
FIG. 12 is a top perspective view of the stent spring in the rolled configuration, according to another example useful for understanding the present disclosure.
FIG. 13 is a top view of the stent spring of FIG. 12.
FIG. 14 is a perspective view of the stent spring of FIG. 12 in the unrolled configuration.
FIG. 15 is a top perspective view of the stent spring, according to another example useful for understanding the present disclosure, wherein the stent spring comprises elastic wires.
FIG. 16 is a top perspective view of the stent spring of FIG. 15 further comprising a connecting band.
FIG. 17 is a top perspective view of another example useful for understanding the present disclosure, the stent spring comprising the elastic wires.
FIG. 18 is a top perspective view of another example useful for understanding the present disclosure, the stent spring comprising the elastic wires.
FIG. 19 is a top perspective view of the stent spring of FIG. 18.
FIG. 20 is a front view of the stent spring comprising a rubber coating according to another example useful for understanding the present disclosure.
FIG. 21 is a perspective view of the stent spring comprising the rubber coating according to another example useful for understanding the present disclosure.
FIG. 22 is a top perspective view of the stent spring of FIG. 20 without the rubber coating.
FIG. 23 is a top perspective view of the stent spring in accordance to another example useful for understanding the present disclosure.
FIG. 24 is a top perspective of the stent spring, according to another example useful for understanding the present disclosure.
FIG. 25 is a top view of the stent spring retained in a compressed stent spring configuration by a compression mechanism.
FIG. 26 is a detail view of the stent spring of FIG. 25 retained in the compressed stent spring configuration by the compression mechanism of FIG. 25.
FIG. 27 is another detail view of the stent spring of FIG. 25 retained in the compressed stent spring configuration by the compression mechanism of FIG. 25.
FIG. 28 is a top perspective view of the stent spring comprising the elastic wires, according to another example useful for understanding the present disclosure.
FIG. 29 is a detail view of the stent spring of FIG. 28.
FIG. 30 is a top perspective view of the stent spring of FIG. 18 further comprising the rubber coating.
FIG. 31 is a detail view of the stent spring of FIG. 30.

### DETAILED DESCRIPTION

The present disclosure can be understood more readily by reference to the following detailed description, examples, drawings, and claims, and the previous and following description. However, before the present devices, systems, and/or methods are disclosed and described, it is to be understood that this disclosure is not limited to the specific devices, systems, and/or methods disclosed unless otherwise specified, and, as such, can, of course, vary. It is also to be understood that the terminology used herein is for the purpose of describing particular aspects only and is not intended to be limiting.

The following description is provided as an enabling teaching of the present devices, systems, and/or methods in its best, currently known aspect. To this end, those skilled in the relevant art will recognize and appreciate that many changes can be made to the various aspects of the present devices, systems, and/or methods described herein, while still obtaining the beneficial results of the present disclosure. It will also be apparent that some of the desired benefits of the present disclosure can be obtained by selecting some of the features of the present disclosure without utilizing other features. Accordingly, those who work in the art will recognize that many modifications and adaptations to the present disclosure are possible and can even be desirable in certain circumstances and are a part of the present disclosure. Thus, the following description is provided as illustrative of the principles of the present disclosure and not in limitation thereof.

As used throughout, the singular forms "a," "an" and "the" include plural referents unless the context clearly dictates otherwise. Thus, for example, reference to "an element" can include two or more such elements unless the context indicates otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

For purposes of the current disclosure, a material property or dimension measuring about X or substantially X on a particular measurement scale measures within a range between X plus an industry-standard upper tolerance for the specified measurement and X minus an industry-standard lower tolerance for the specified measurement. Because tolerances can vary between different materials, processes and between different models, the tolerance for a particular measurement of a particular component can fall within a range of tolerances.

As used herein, the terms "optional" or "optionally" mean that the subsequently described event or circumstance can or cannot occur, and that the description includes instances where said event or circumstance occurs and instances where it does not.

The word "or" as used herein means any one member of a particular list and also includes any combination of members of that list. Further, one should note that conditional language, such as, among others, "can," "could," "might," or "may," unless specifically stated otherwise, or otherwise understood within the context as used, is generally intended to convey that certain aspects include, while other aspects do not include, certain features, elements and/or steps. Thus, such conditional language is not generally intended to imply that features, elements and/or steps are in any way required for one or more particular aspects or that one or more particular aspects necessarily include logic for deciding, with or without user input or prompting, whether these features, elements and/or steps are included or are to be performed in any particular aspect.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific aspect or combination of aspects of the disclosed methods.

Disclosed in the present application is a stent for repairing a pipe, and associated methods, systems, devices, and various apparatus. Example aspects of the stent can be oriented in an expanded configuration and a compressed configuration. The stent can comprise a stent spring and a seal. Example aspects of the stent spring can define a tubular mesh structure comprising one or more strands. It would be understood by one of skill in the art that the disclosed stent is described in but a few exemplary aspects among many. No particular terminology or description should be considered limiting on the disclosure or the scope of any claims issuing therefrom.

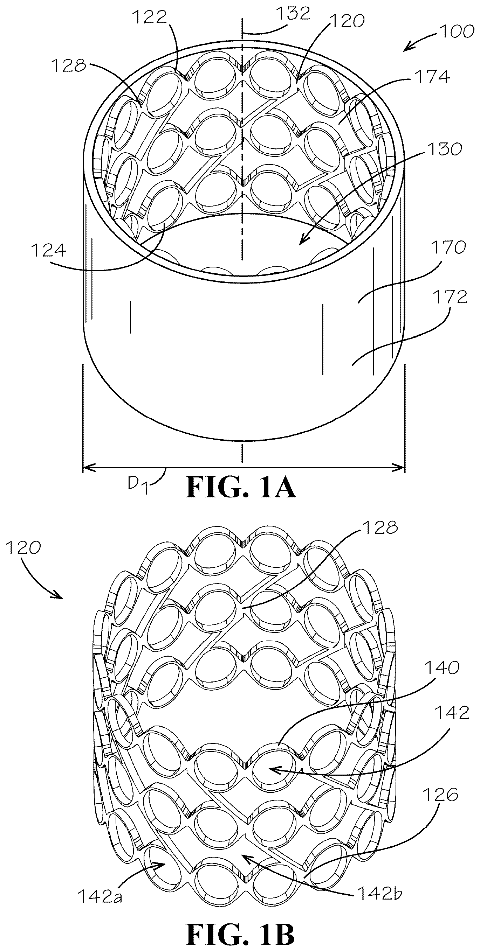

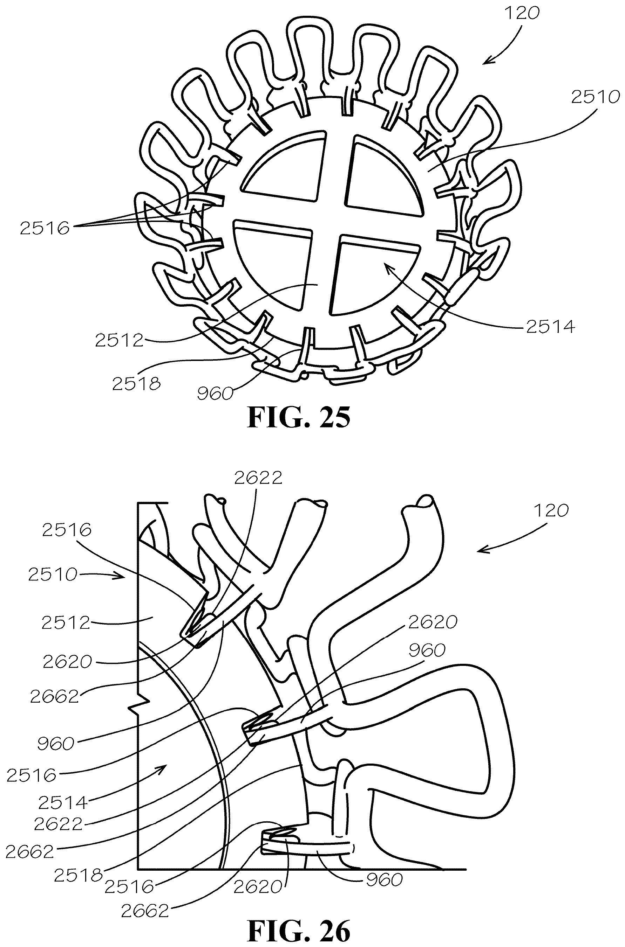

Figure 1A illustrates a first aspect of a stent 100 according to the present disclosure. As shown, the stent 100 can comprise a stent spring 120 and a seal 170. Example aspects of the stent spring 120 can define a spring force and can be expandable and compressible, such that the stent spring 120 can be oriented in an expanded stent spring configuration, as shown in Figure 1A, and a compressed stent spring configuration, as shown in Figure 25. As such, the stent 100 itself can also be oriented in an expanded configuration and a compressed configuration. According to example aspects, the stent 100 can be expanded within a pipe (not shown) such that the seal 170 can engage an inner wall (not shown) of the pipe where a crack or other damage is present, in order to create a watertight seal between the stent 100 and the inner wall of the pipe to prevent leaking at the damage site.

As shown in Figure 1A, the stent spring 120 can bias the stent 100 to the expanded configuration. In the depicted aspect, the stent spring 120 can be formed as a substantially tubular mesh structure defining opposing open ends (e.g. a top end 122 and a bottom end 124). The stent spring 120 can further define an outer surface 126 (shown in Figure 1B) and an opposite inner surface 128. The inner surface 128 can define a void 130. The void 130 can extend between the open top and bottom ends 122,124 of the stent spring 120, and can allow fluid to pass therethrough when the stent 100 is received in the pipe. A central axis 132 can extend substantially through a center of the void 130, as shown. According to example aspects, the stent spring 120 can be formed from a spring material. For example, the stent spring 120 can comprise a metal material, such as stainless steel, spring steel, aluminum, nitinol, cobalt chromium, or any other suitable material. In other aspects, the stent spring 120 can be formed from a plastic material, such as, for example, nylon, POM (polyoxymethylene), or PVC (polyvinyl chloride). In still another aspect, the stent spring 120 can be formed from a carbon fiber material. Optionally, the material can be an NSF certified material that can comply with various public health safety standards. For example, in some aspects, the material can be approved as safe for use in drinking-water applications. Moreover, in some aspects, the stent spring 120 can comprise a coating, such as, for example, a rubber or liquid metal coating. The coating can improve mechanical properties of the stent spring 120. For example, the coating can improve the tensile strength of the stent spring 120 by providing a flexible and/or springy outer layer. In some aspects, the coating can also be corrosion resistant, or a separate coating can be applied for corrosion resistance. For example, a corrosion resistant coating can comprise a zinc-nickel material, phosphate, electrophoretic paint (e-coating), polyester, fusion-bonded epoxy (FBE), or any other suitable corrosion resistant material.

Example aspects of the seal 170 can be formed as a continuous, tubular sleeve structure, as shown, and can define an outer surface 172 and an inner surface 174. In the present aspect, the outer surface 172 of the seal 170 can define a stent diameter D₁ of the stent 100. Example aspects of the seal 170 can comprise a flexible and compressible material, such as, for example, neoprene. In other aspects, the seal 170 can be formed from another synthetic rubber material such as EPDM rubber, natural rubber, foam, epoxy, silicone, a resin-soaked cloth, or any other suitable flexible material for providing a watertight seal between the stent 100 and the inner wall of the pipe. According to example aspects, the seal 170 can wrap around a circumference of the stent spring 120, and the inner surface 174 of the seal 170 can engage the outer surface 126 of the stent spring 120. In a particular aspect, the seal 170 can cover the entire outer surface 126 of the stent spring 120, as shown. However, in other aspects, the seal 170 can cover only a portion of the outer surface 126 of the stent spring 120. In still other aspects, the seal 170 may not wrap entirely around the circumference of the stent spring 120. In the present aspect, the seal 170 can fit snugly on the stent spring 120. In some aspects, the seal 170 can be coupled to the stent spring 120 by a fastener (not shown), such as, for example, stitching, adhesives, ties, or any other suitable fastener known in the art.

In the expanded configuration of the stent 100, as shown in Figure 1A, the spring force of the stent spring 120 can bias the stent spring 120 and the seal 170 radially outward relative to the central axis 132, such that each of the stent spring 120 and seal 170 can define relatively concentric tubular shapes, as shown. In the expanded configuration, the stent 100 can define its largest possible stent diameter D₁. In some aspects, in the expanded configuration, the stent diameter D₁ can be slightly greater than an inner pipe diameter as defined by the inner wall of the pipe to aid in retaining the stent 100 against the inner wall.

In the compressed configuration, a compression force (i.e., a pushing force, a pulling force, or any other suitable force) can be applied to the stent 100 by a compression mechanism to bias the stent 100, including the stent spring 120 and the seal 170, to the compressed configuration. Various example aspects of such the compression mechanism are described through the present application, including, for example, an internal compression disc 2510 (shown in Figure 25). The compression force can overcome the spring force, and the seal 170 and stent spring 120 can compress or fold radially inward towards the void 130 to define a smaller stent diameter D₁ and a smaller overall stent volume than in the expanded configuration. The reduced stent diameter D₁ and stent volume in the compressed configuration can allow for easier insertion of the stent 100 into the pipe or a pipeline (not shown) and easier navigation of the stent 100 through the pipe or pipeline. When the compression force is removed or reduced to less than the spring force, the stent spring 120 can bias the stent 100 back to the expanded configuration.

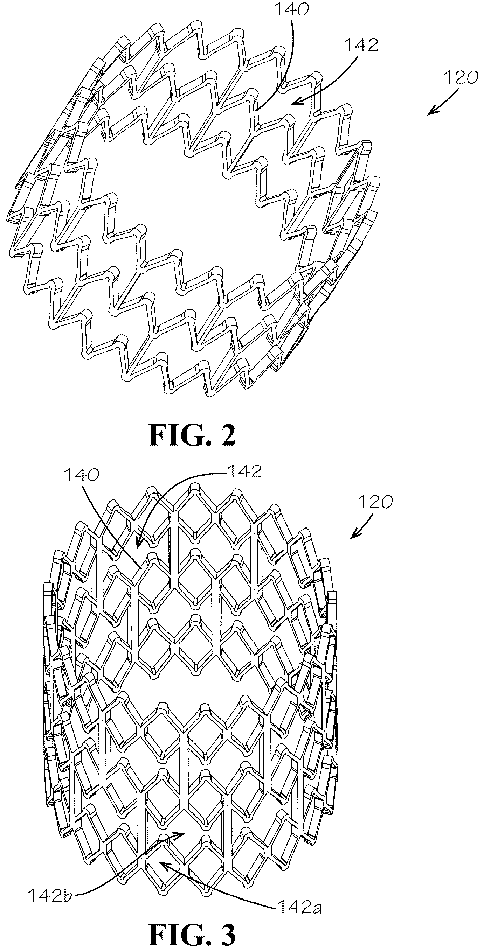

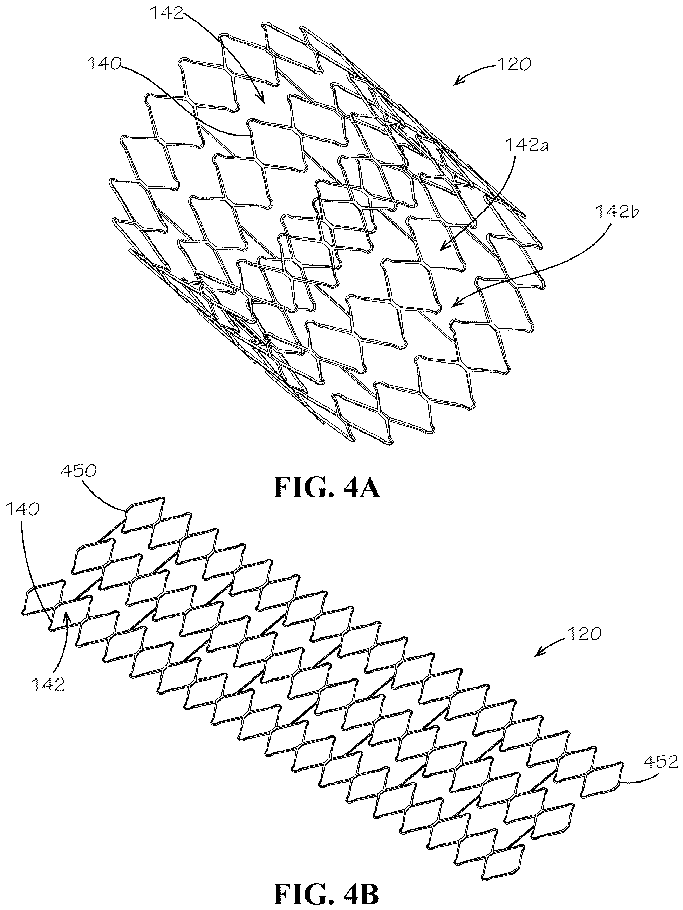

Figure 1B illustrates the stent spring 120 of Figure 1A with the seal 170 (shown in Figure 1A) removed for full visibility of the of the stent spring 120. As shown, the tubular mesh structure of the stent spring 120 can comprise one or more strands 140 arranged to define a plurality of openings 142 therebetween. In the present aspect, as shown, a plurality of the openings 142a can define a substantially circular shape, while other openings 142b can define a shape that is substantially that of a pair of conjoined diamonds. In other aspects, the openings 142 can define any other suitable shape(s), some examples of which are described below. According to example aspects, the mesh structure of the stent spring 120 can be laser cut, chemically etched, or stamped from a sheet of material (e.g., a sheet of metal). In other aspects, the mesh structure of the stent spring 120 can be formed by stereolithography (e.g., 3D printing), or by any other suitable manufacturing method suitable for forming a mesh structure. In some example aspects, the stent spring 120 can be oriented in a rolled configuration for use, as shown, and an unrolled configuration, as shown in Figure 4B. In example aspects, the stent spring 120 can be manufactured in the unrolled configuration, and rolled into the rolled configuration thereafter for use. Figures 2 and 3 each illustrate an additional example aspect of the stent spring 120 in the rolled configuration. As shown in the aspect of Figure 2, some or all of the openings 142 can substantially define an M-shape. As shown in the aspect of Figure 3, some of the openings 142a can substantially define a diamond shape, and some other openings 142b can substantially define a series of conjoined diamond and half-diamond shapes.

Figure 4A illustrates the stent spring 120 in the rolled configuration, according to another aspect of the present disclosure, and Figure 4B illustrates the stent spring 120 of Figure 4A in the unrolled configuration. As shown in Figure 4A, some of the openings 142a can substantially define a diamond shape, and some other openings 142b can substantially define a conjoined series of diamond and partial-diamond shapes. As shown, in the unrolled configuration, the stent spring 120 can be substantially flat and can define a first end 450 and an opposing second end 452. According to example aspects, the mesh structure of the stent spring 120 can be manufactured in the unrolled configuration, for example, by laser cutting or sterolithography. The stent spring 120 can then be rolled into the rolled configuration. To retain the stent spring 120 in the rolled configuration, the first end 450 of the stent spring 120 can be spot welded, riveted, or otherwise attached by any suitable attachment method, to the second end 452. In other aspects, the first end 450 of the stent spring 120 can be attached to the second end 452 by a fastener, such as, for example, one or more nut and bolt assemblies, adhesives, clips, snaps, ties, or any other suitable fastener or combination of fasteners know in the art. Furthermore, according to example aspects, the rolled stent spring 120 (or in other aspects, the unrolled stent spring 120) can be heat treated to harden the stent spring 120. In one example aspect, the stent spring 120 can be hardened to between about 40-45 HRC, for example and without limitationcircular.

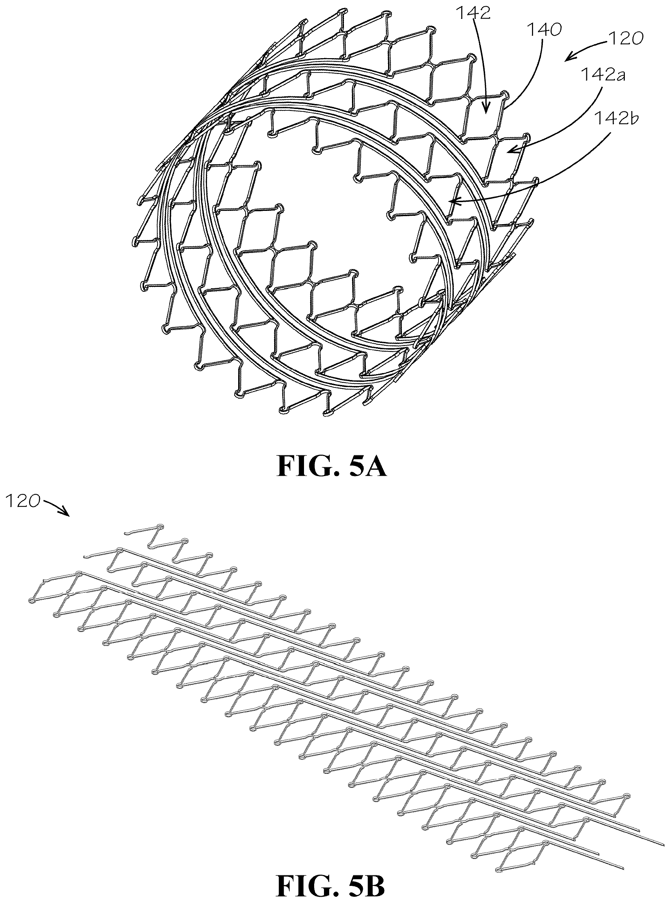

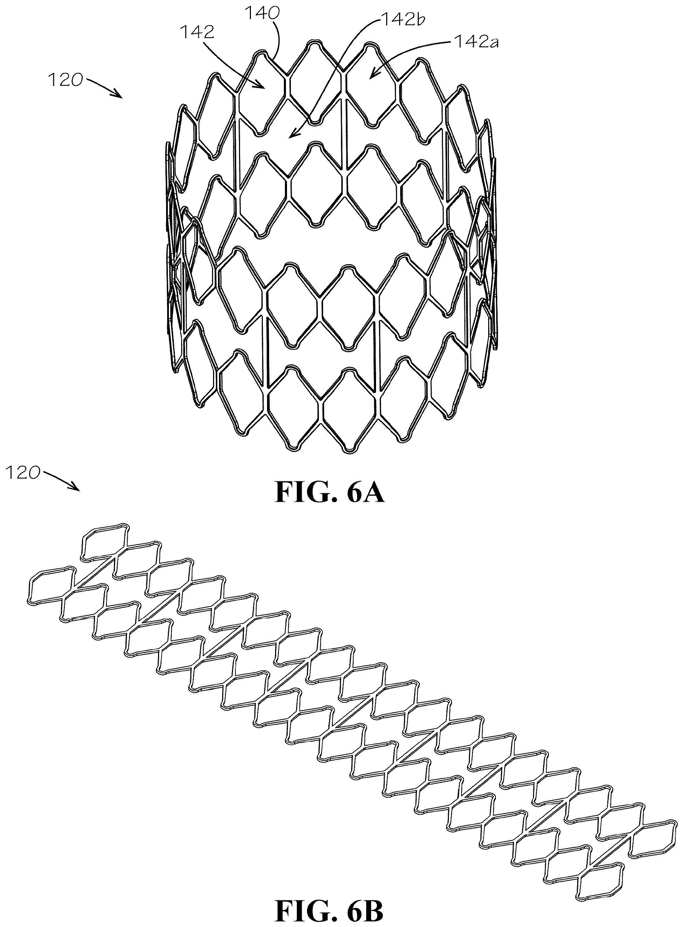

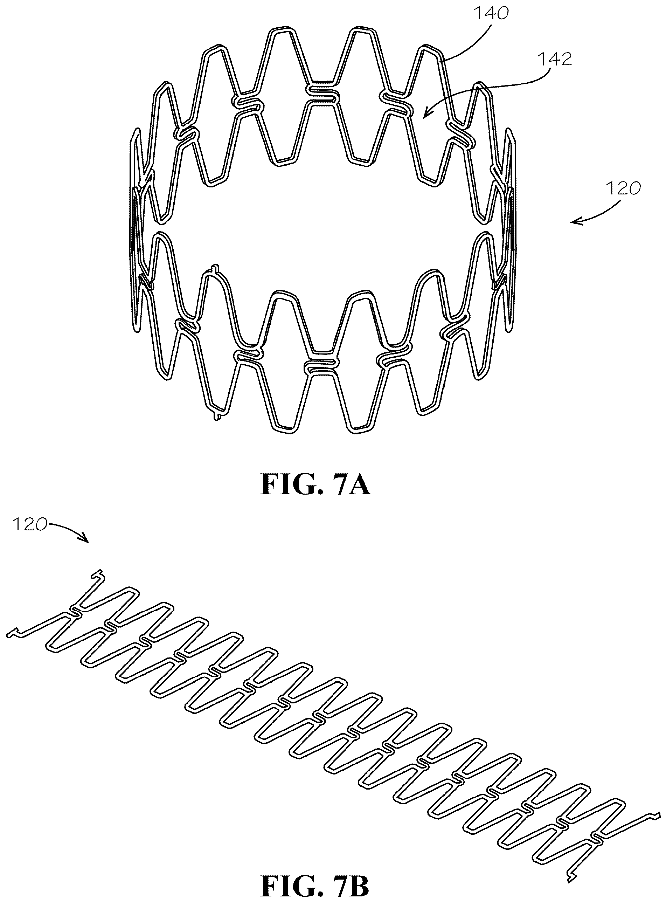

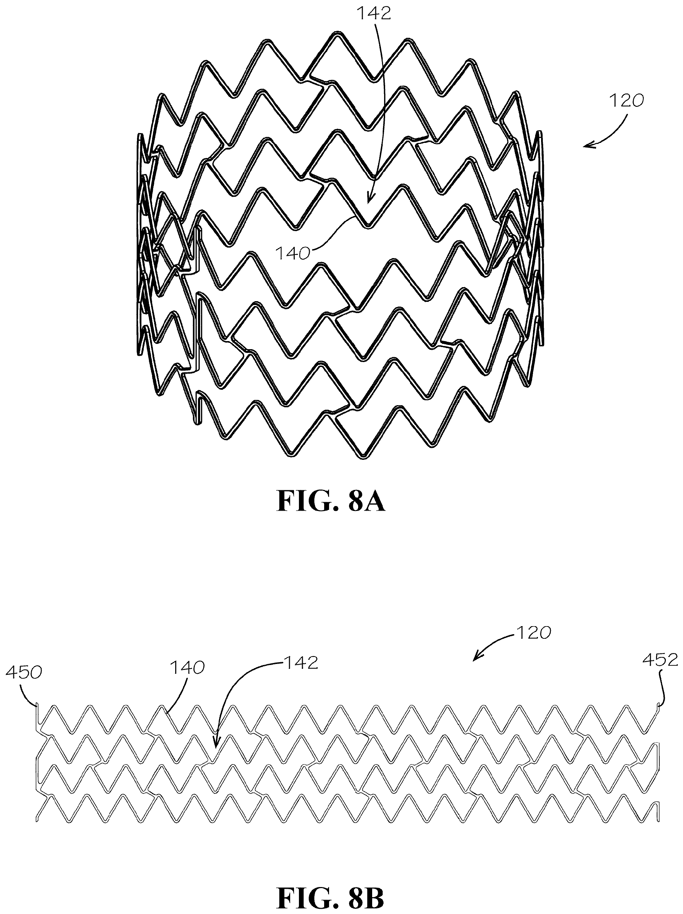

Figure 5A illustrates the stent spring 120 in the rolled configuration, according to another aspect of the present disclosure, and Figure 5B illustrates the stent spring 120 of Figure 5A in the unrolled configuration. Referring to Figure 5A, in the present aspect, some of the openings 142a can substantially define a diamond shape, and some other openings 142b can substantially define a pair of half-diamond shapes connected by an elongated rectangular shape. Figure 6A illustrates the stent spring 120 in the rolled configuration, according to yet another aspect of the present disclosure, and Figure 6B illustrates the stent spring 120 of Figure 6A in the unrolled configuration. Referring to Figure 6A, in the present aspect, some of the openings 142a can substantially define a diamond shape, and some other openings 142b can substantially define a series of diamond and half-diamond shapes. Figure 7A illustrates still another aspect of the stent spring 120 in the rolled configuration, and Figure 7B illustrates the stent spring 120 of Figure 7A in the unrolled configuration. In the present aspect, the openings 142 can substantially define an elongated hexagonal shape. Figure 8A illustrates the stent spring 120 in the rolled configuration, according to a further aspect of the present disclosure, and Figure 8B illustrates the stent spring 120 of Figure 8A in the unrolled configuration. In the present aspect, the openings 142 can substantially define a chevron pattern.

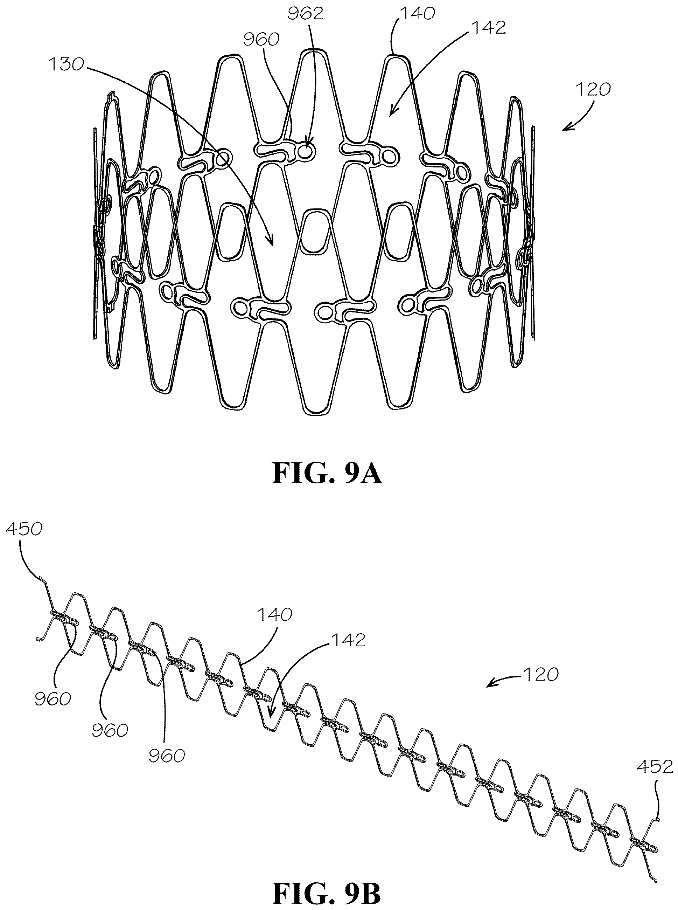

Figure 9A illustrates the stent spring 120 in the rolled configuration, according to yet another aspect of the present disclosure, and Figure 9B illustrates the stent spring 120 of Figure 9A in the unrolled configuration. In the present aspect, the openings 142 can substantially define an elongated hexagonal shape. Furthermore, in the present aspect, the stent spring 120 can comprise a spring steel material. Example aspects can be coated with a rubber or liquid metal material, zinc-nickel material, phosphate, electrophoretic paint (e-coating), polyester, or fusion-bonded epoxy (FBE), as described above. In other aspects, the stent spring 120 can comprise a stainless steel material, or any other suitable spring material. As shown, example aspects of the stent spring 120 can comprise one or more tabs 960, each defining a tab opening 962 therethrough. The tabs 960 can be bent inward towards the void 130 and the compression mechanism can engage the tabs 960 to compress the stent spring 120 to the compressed stent spring configuration. In a first example aspects, a cable (not shown), or other fastening device, can pass through the tab opening 962 of each of the tabs 960 and can be tightened to contract the stent 100 (shown in Figure 1A) to the compressed configuration.

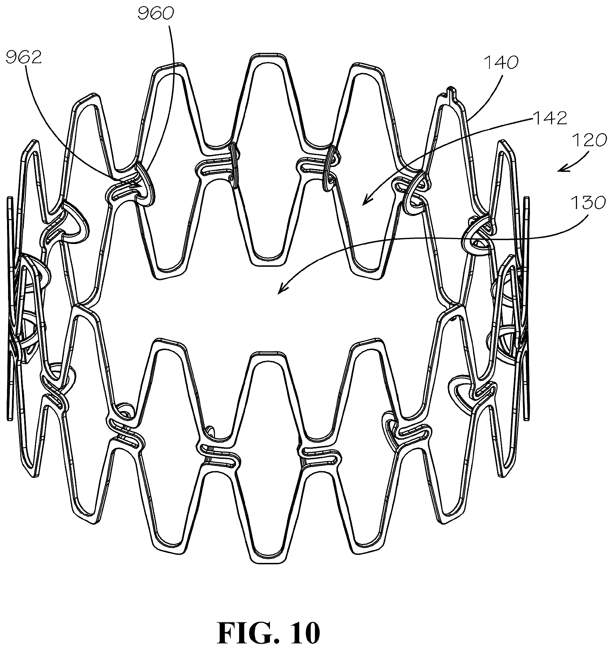

Figure 10 illustrates still another aspect of the stent spring 120 the rolled configuration. In the present aspect, the openings 142 can substantially define an elongated hexagonal shape. Furthermore, in the present aspect, the stent spring 120 can comprise a carbon fiber material. As shown, the stent spring 120 comprises the tabs 960 extending radially inward towards the void 130. In the present aspect, the tabs 960 can be formed extending inward rather than having to be bent inwards, as may be required by the aspect of Figure 9A. Each of the tabs 960 can define one of the tab openings 962 therethrough. As described above, in example aspects, a cable (not shown) can pass through the tab opening 962 of each of the tabs 960 and can be tightened to contract the stent 100 (shown in Figure 1A) to the compressed configuration through tension in the cable. The cable can be cut to release the tension force on the stent 100 and to allow the stent spring 120 to return to the expanded stent spring configuration, thus biasing the stent 100 to the expanded configuration. In other aspects, the stent 100 can be compressed by another compression or contraction mechanism, such as a compression sleeve or tube, a dissolvable wire, or any other suitable mechanisms known in the art. In an aspect comprising a dissolvable wire, the wire can be dissolved by electricity, chemicals, water, or any other suitable dissolving mechanism. In still another aspect, the compression mechanism can be a hose clamp. In some aspects, the hose clamp or other compression mechanism can comprise a worm drive.

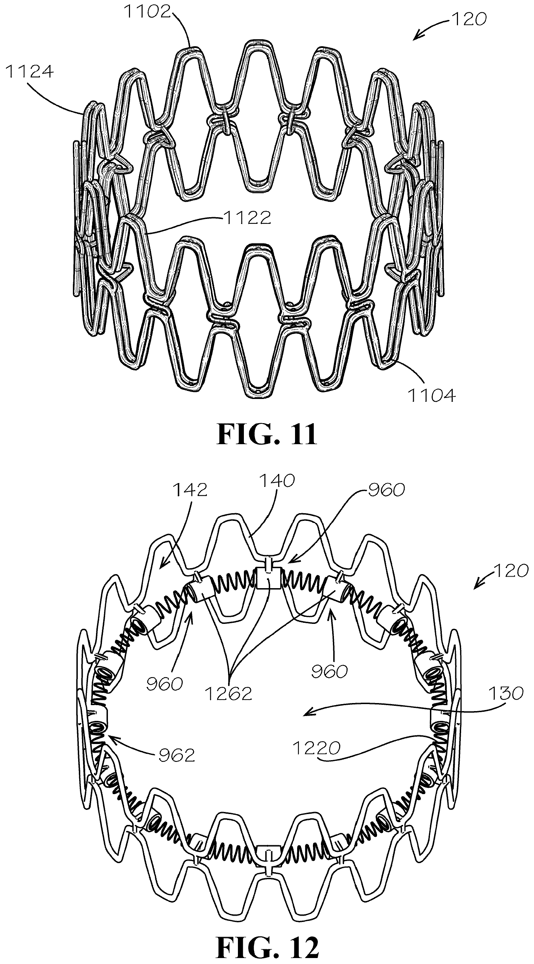

Figure 11 illustrates an aspect of the present disclosure of the stent spring 120 in the rolled configuration. As shown, the present stent spring 120 can comprise an inner stent spring 1122 aligned and connected with an outer stent spring 1124 to provide increased stiffness of the stent spring 120, while maintaining flexibility of the stent spring 120. Each of the inner stent spring 1122 and outer stent spring 1124 of the present aspect can be substantially similar in shape to the stent spring 120 illustrated in Figure 10; however, in other aspects, the inner and outer stent springs 1122,1124 can be differently shaped. In one example aspect, the inner and outer stent springs 1122,1124 can be formed from carbon fiber, and in another example aspect, the inner and outer stent springs 1122,1124 can be formed from nylon. In other aspects, the inner and outer stent springs 1122,1124 can be formed from any suitable material, including but not limited to stainless steel, spring steel, aluminum, nitinol, cobalt chromium, POM (polyoxymethylene), and PVC (polyvinyl chloride). According to example aspects, the inner and outer stent springs can be joined together at a plurality of upper bends 1102 and lower bends 1104 thereof, as shown.

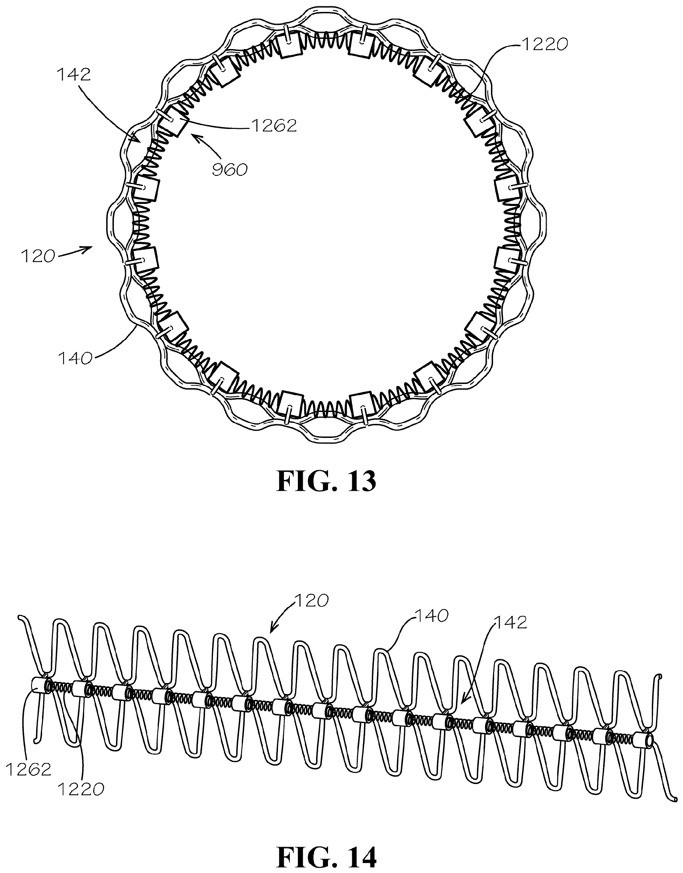

Figures 12 and 13 illustrates an example aspect of the stent spring 120 in the rolled configuration, wherein the tabs 960 are formed as hollow cylindrical structures 1262 each defining the tab opening 962 extending therethrough. In the present aspect, a coil spring 1220 can extend through the tab openings 962, as shown. The coil spring 1220 can define a coil spring force. In example aspects, like the stent spring 120, the coil spring 1220 can be compressed in the compressed stent spring configuration and can be expanded in the expanded stent spring configuration. As described above, in the compressed stent spring configuration, a compression force (e.g. a pushing force, tension or pulling force, or any other suitable force) can be applied to the stent 100 (shown in Figure 1A). The compression force can overcome the spring force of the stent spring 120 and the coil spring force of the coil spring 1220, and the stent spring 120, coil spring 1220, and seal 170 (shown in Figure 1A) can be compressed or folded radially inward towards the void 130. When compressed, the stent 100 can define a smaller stent diameter D₁ (shown in Figure 1A) and a smaller overall stent volume than in the expanded configuration. When the compression force is removed or reduced to less than the spring force and coil spring force, both of the stent spring 120 and the coil spring 1220 can assist in biasing the stent 100 fully back to the expanded configuration. As such, in instances where one of the stent spring 120 and coil spring 1220 may not bias the stent 100 fully back to the expanded configuration on its own, the other of the stent spring 120 and coil spring 1220 can assist in further biasing the stent 100 towards the expanded configuration. Moreover, as shown in Figure 13, example aspects of the stent spring 120 can be formed from a Windform^{®} material, such as, for example, a Windform^{®} SP material. The Windform SP material is a carbon fiber reinforced composite polyamide material, which can be durable, insulating, and water resistant. Figure 14 illustrates the stent spring 120 of Figures 12 and 13 in the unrolled configuration.

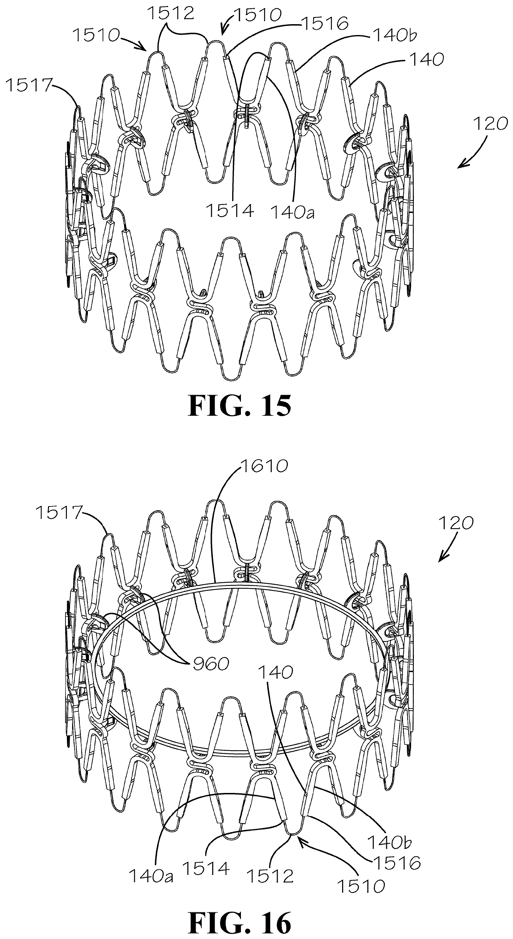

Figures 15 and 16 illustrates an example aspect of the stent spring 120 in the rolled configuration, according to another aspect of the present disclosure. The stent spring 120 can be similar to the stent spring 120 illustrated Figure 10. However, as shown, the stent spring 120 of the present aspect can further comprise a wire or wires 1510 connected to one or more of the strands 140 of the stent spring 120. In one example aspect, the wires 1510 can be a plurality of Nitinol super-elastic wires 1512, which can be configured to provide added flexibility to the stent spring 120. In example aspects, each of the Nitinol super-elastic wires 1512 can define a first end 1514, a second end 1516, and a middle section 1517 extending therebetween. The first end 1514 can be received within a first groove (not shown) formed within a corresponding first strand 140a, and the second end 1516 can be received within a second groove (not shown) of an adjacent second strand 140b.

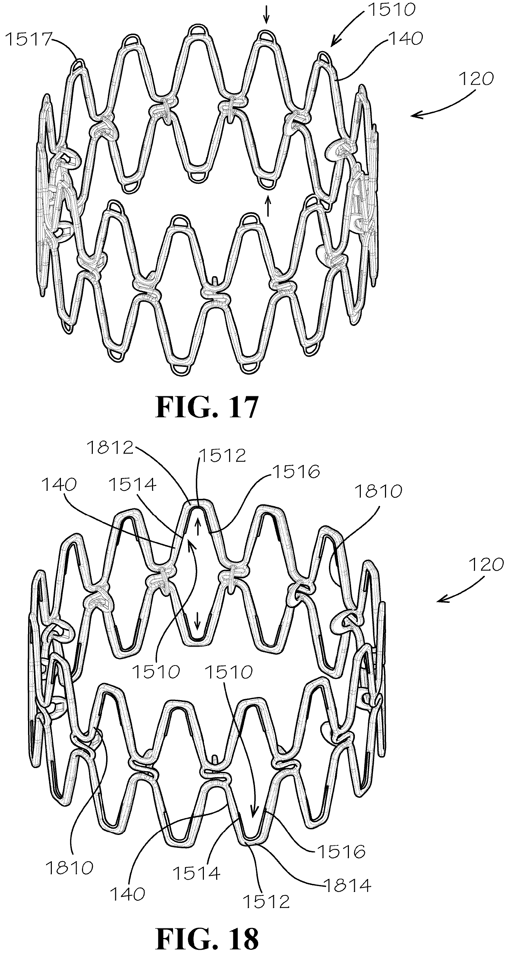

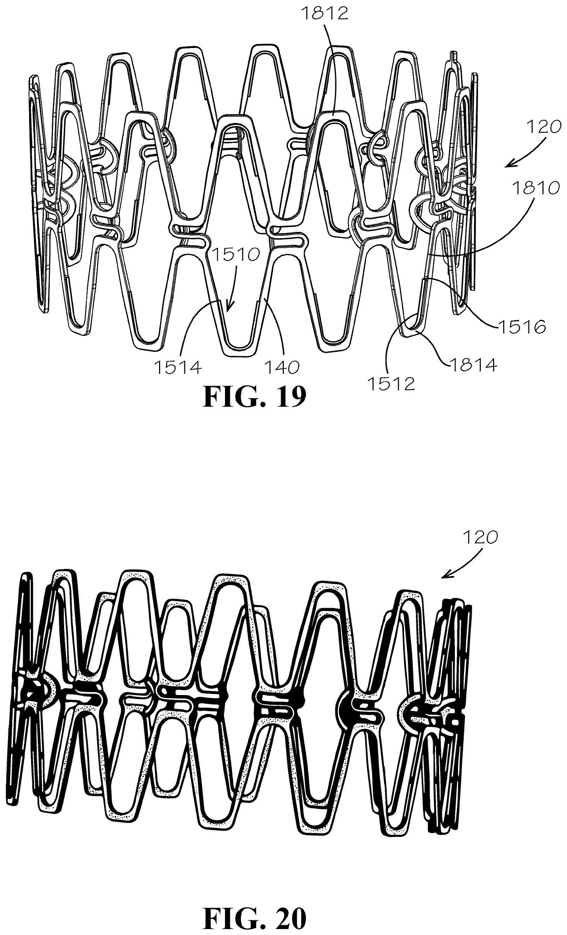

As shown in Figure 16, in some aspects, the compression mechanism can be a connecting band 1610. The connecting band 1610 can engage each of the tabs 960 of the stent spring 120 to retain the stent spring 120 in the compressed stent spring configuration while the wires 1510 are assembled with the stent spring 120. Furthermore, in the present aspect, the middle section 1517 of each wire 1510 can be substantially exposed. However, in other aspects, the wires 1510 can be more fully received within the strands 140 of the stent spring 120, such that a lesser portion of the middle section 1517 is exposed, as depicted in Figure 17, and in still other aspects, the wires 1510 can be completely received within the strands 140. Figures 18 and 19 illustrates another aspect, wherein each of the wires 1510 can be positioned on an inner periphery 1810 of the stent spring 120 proximate to an upper bend 1812 or lower bend 1814 thereof. In one aspect, the wires 1510 can be connected to the stent spring 120 by an adhesive, or other fastener, and the first and second ends 1514,1516 of the wires 1510 do not extend into the strands 140. However, in other aspects, the first and second ends 1514,1516 of each of the wires 1510 can engage the first and second grooves (not shown) formed in a corresponding strand 140 to connect the wire 1510 to the stent spring 120.

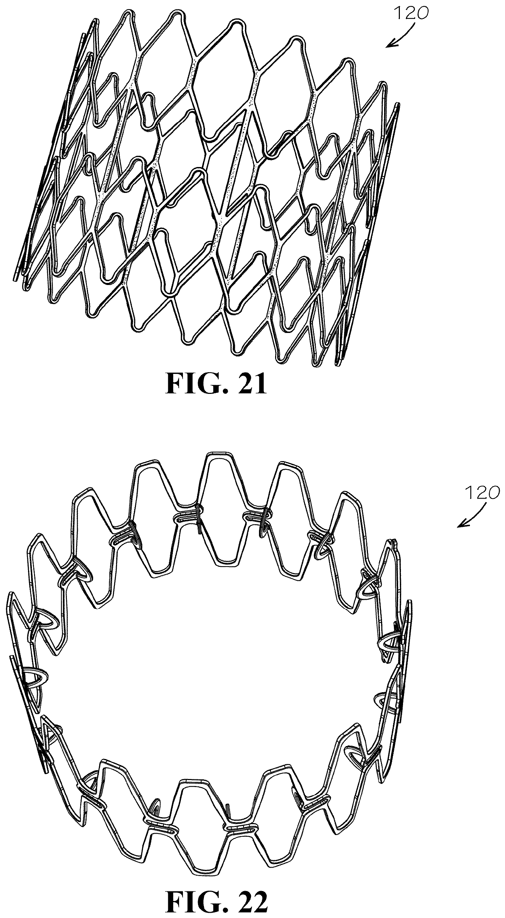

Example aspects of the stent spring 120 can comprise a coating, such as, for example, a rubber coating. For example, as shown in the aspect of Figure 20, the stent spring 120 can be coated in a Plasti Dip^{®} coating. A Plasti Dip^{®} coating is a synthetic rubber coating that can be applied by spraying, brushing, dipping, or the like, and which can be configured to air dry. The Plasti Dip^{®} material can be non-slip, flexible, durable, and insulating material in some aspects. In another example aspect, as shown in Figure 21, the stent spring 120 can be coated in a Flex Seal^{®} coating. The Flex Seal^{®} coating is a synthetic rubber coating similar to the Plasti Dip^{®} coating. The Flex Seal^{®} coating can be applied by pouring, rolling, dippy, spraying, or the like, and can be durable, flexible, insulating, and water resistant. In other aspects, the coating can be any other suitable coating known in the art. Example aspects of the coating can be flexible and can improve the flexibility of the stent spring 120. In some example aspects, the coating can also be a non-slip coating that can improve the grip of the stent spring 120 on the seal 170 (shown in Figure 1A), the pipe (not shown), or any other component engaged by the stent spring 120. Figure 22 illustrates the stent spring 120 of Figure 20 without the Plasti Dip^{®} coating applied.

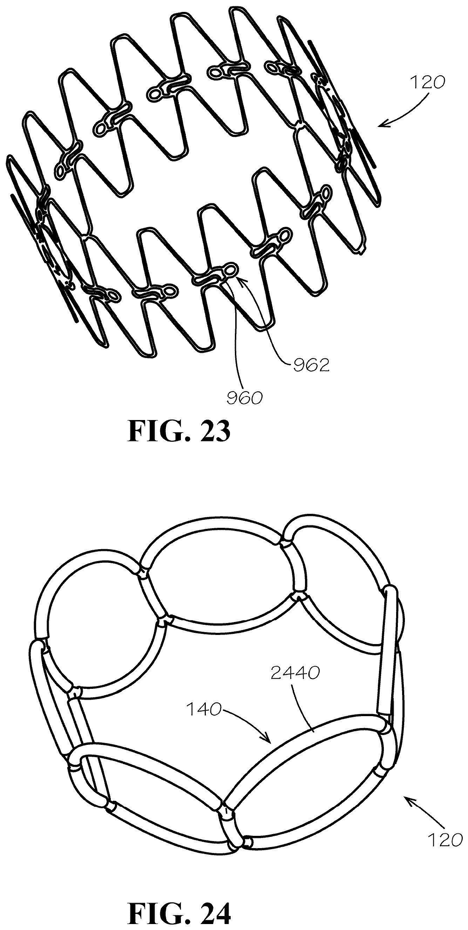

Figure 23 illustrates another example aspect of the stent spring 120 that can be substantially similar to the stent spring 120 of Figure 9A. However, in the present aspect, as shown, the tabs 960 can define larger tab openings 962 than the tab openings 962 shown in Figure 9A. The larger tab openings 962 can accommodate for a larger or different compression mechanism for compressing the stent 100 (shown in Figure 1A). Figure 24 illustrates still another example aspect of the stent spring 120, wherein the strands 140 of the stent spring 120 can be a plurality of connected, substantially circular, resilient and flexible strands 2440, as shown. The flexibility of the strands 140 can allow the stent spring 120 to be compressed to the compressed stent spring configuration, and the resiliency of the strands 140 can bias the stent spring 120 from the compressed stent spring configuration to the expanded stent spring configuration.

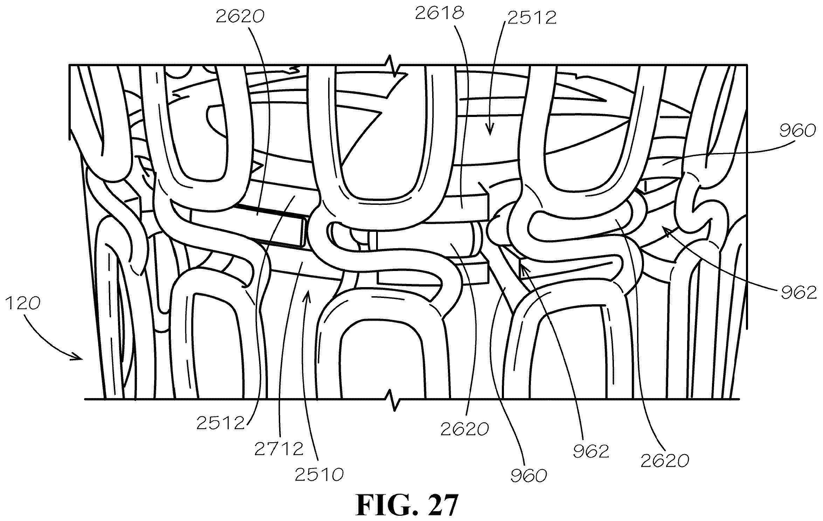

According to example aspects, the stent spring 120 can be compressed by the compression mechanism, as described above. For example, in a particular aspect, the compression mechanism can be an internal compression disc 2510 as illustrated in Figure 25. According to example aspects, the compression disc 2510 can engage each of the tabs 960 of the stent spring 120 to pull the stent spring 120 radially inward and to retain the stent spring 120 in the compressed stent spring configuration. In the present aspect, the compression disc 2510 can comprise an upper disc 2512 and a lower disc 2712 (shown in Figure 27) connected to the upper disc 2512. Disc openings 2514 can be formed in each of the upper and lower discs 2512,2712 to allow for fluid flow therethrough. Furthermore, one or more disc slots 2516 can be formed at an outer side edge 2518 of the compression disc 2510.

Referring to Figures 26 and 27, the compression disc 2510 can further comprise a plurality of connectors 2620 generally received between the upper disc 2512 and lower disc 2712 and proximate to the outer side edge 2518 of the compression disc 2510. A head 2622 of each of the connectors 2620 can be configured to extend into a corresponding one of the disc slots 2516. To mount the stent spring 120 to the compression disc 2510 in the compressed stent spring configuration, an inner end 2662 of each of the tabs 960 can be pushed past the head 2622 of the corresponding connector 2620 and into the corresponding disc slot 2516, such that the head 2622 of each connector 2620 extends through the tab opening 962 (shown in Figure 9A) of the corresponding tab 960. To move the stent spring 120 to the expanded stent spring configuration, the compression disc 2510 can be slid axially relative to the central axis 132 (shown in Figure 1A). The tabs 960 of the stent spring 120 can be pushed past the heads 2622 of the corresponding connectors 2620, such that each of the connectors 2620 can be disengaged from the corresponding tab opening 962, and the compression disc 2510 can be disengaged from the stent spring 120. With the compression disc 2510 disengaged from the stent spring 120, the spring force of the stent spring 120 can bias the stent 100 (shown in Figure 1A) to the expanded configuration.

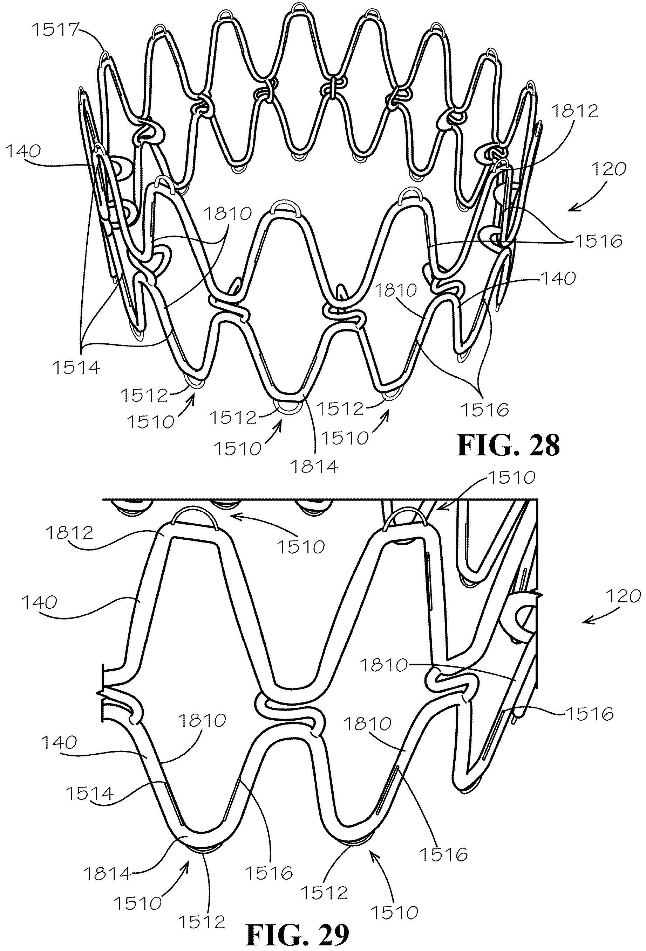

Figures 28 and 29 illustrate another aspect of the stent spring 120 comprising the wires 1510 (e.g., the Nitinol super-elastic wires 1512). The stent spring 120 of the present aspect can be similar to the stent spring 120 of Figure 17, wherein the first end 1514 of each wire 1510 can be received through the first groove (not shown) formed within one of the strands 140, and the second end 1516 of each wire 1510 can be received within the second groove (not shown) formed in the same strand 140. Each wire 1510 can be oriented proximate to one of the upper bends 1812 or lower bends 1814 of the stent spring 120, as shown. In the present aspect, the first and second ends 1514,1516 of each of the wires 1510 can pass through the corresponding first and second grooves, respectively, and can abut the inner periphery 1810 of the stent spring 120 proximate to the corresponding upper or lower bend 1812,1814, as shown. The middle section 1517 can be exposed.

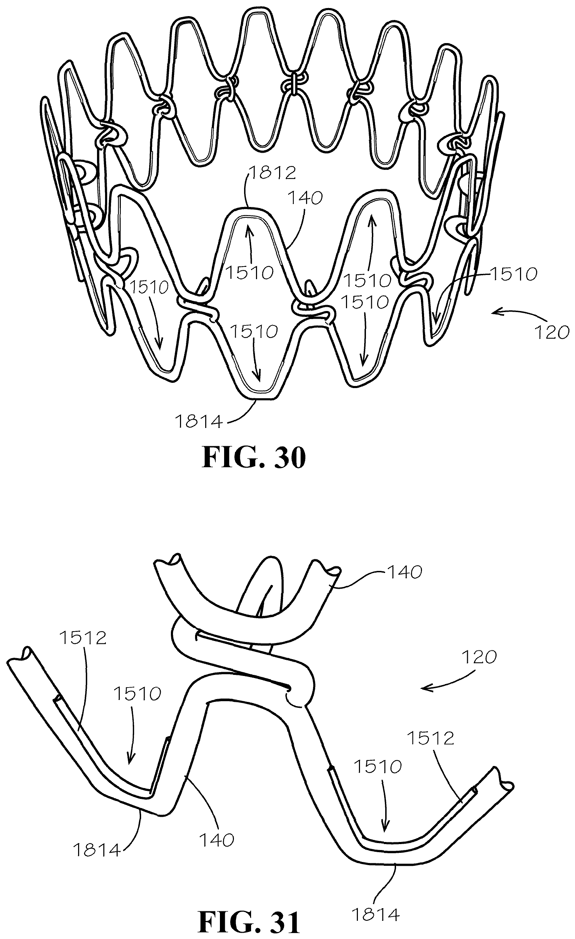

Figures 30 and 31 illustrate the stent spring 120 of Figures 18 and 19 dipped in the rubber coating, such as, for example, the Plasti Dip^{®} coating or the Flex Seal^{®} coating, as described above with reference to Figures 20, 21, and 22.

In one exemplary aspect, a stent spring for repairing a pipe can comprise a substantially tubular mesh structure defining a void, the void can define a central axis, the mesh structure can comprise one or more strands, and the one or more strands can define a plurality of openings. The stent spring can be configurable in an expanded stent spring configuration and a compressed stent spring configuration. The stent spring can further comprise a tab extending radially inward from the mesh structure into the void, and the tab can define a tab opening.

In a further exemplary aspect, the stent spring can further comprise a compression mechanism configured to engage the tab to bias the stent spring to the compressed stent spring configuration. In a further exemplary aspect, the compression mechanism can comprise a cable configured to extend through the tab opening of the tab. In a further exemplary aspect, the compression mechanism can comprise a compression disc, wherein the compression disc can define a disc slot formed in an outer side edge of the compression disc, and a connector, wherein the connector can comprise a head and the head can extend into the disc slot. In a further exemplary aspect, the tab can extend into the disc slot, and the head can extend through the tab opening. In a further exemplary aspect, the stent spring can further comprise one or more elastic wires connected to the one or more strands, wherein the one or more elastic wires can be configured to increase a flexibility of the stent spring. In a further exemplary aspect, the stent spring can further comprise a coil spring extending through the tab opening, wherein the coil spring can be configured to assist in biasing the stent spring to the expanded stent spring configuration. In a further exemplary aspect, the stent spring can further comprise a flexible coating on the one or more strands, wherein the flexible coating can comprise a synthetic rubber material. In a further exemplary aspect, the stent spring can comprise an inner stent spring connected to an outer stent spring, the inner stent spring can comprise the mesh structure and the tab, and the outer stent spring can be configured to increase a stiffness of the inner stent spring.

In another exemplary aspect, a stent spring for repairing a pipe can comprise a substantially tubular mesh structure comprising one or more strands, and the one or more strands can comprise a spring material. The stent spring can be expandable and compressible between an expanded stent spring configuration and a compressed stent spring configuration. The stent spring can further comprising an elastic wire connected to the one or more strands, wherein the elastic wire can be configured to increase a flexibility of the stent spring.

In a further exemplary aspect, the elastic wire can define a first end and a second end, the first end of the elastic wire can engage a first one of the strands, and the second end of the elastic wire can engage a second one of the strands. In a further exemplary aspect, the first one of the strands can define a first groove, the second one of the strands can define a second groove, the first end can be received within the first groove, and the second end can be received within the second groove. In a further exemplary aspect, the elastic wire can define a middle section between the first end and second end, and at least a portion of the middle section can be exposed. In a further exemplary aspect, the elastic wire can be positioned on an inner periphery of the stent spring. In a further exemplary aspect, the one or more strands can define at least one of an upper bend and a lower bend, and the elastic wire can be positioned proximate to the at least one of an upper bend and a lower bend. In a further exemplary aspect, a first end of the elastic wire can extend through a first groove of the mesh structure and abut an inner periphery of the stent spring, a second end of the elastic wire can extend through a second groove of the mesh structure and abut an inner periphery of the stent spring, and a middle section of the elastic wire can be exposed. In a further exemplary aspect, the one or more strands can define a plurality of openings. In a further exemplary aspect, the stent spring can further comprise a tab extending radially inward from the mesh structure and a compression mechanism engaging the tab to retain the stent spring in the compressed stent spring configuration.

In another exemplary aspect, a method for retaining a stent in a compressed configuration can comprise providing a stent, wherein the stent can comprise a stent spring, a seal, and a tab extending radially inward from the stent spring. The method can further comprise biasing the stent to a compressed configuration and engaging the tab with a compression mechanism to retain the stent in the compressed configuration.

In a further exemplary aspect, engaging the tab with a compression mechanism can comprise inserting the tab into a disc slot of the compression mechanism and engaging a tab opening of the tab with a connector of the compression mechanism.

In another exemplary aspect, a stent can comprise a stent spring and a seal wrapped around a circumference of the stent spring, the stent spring can define a substantially tubular mesh structure, the stent can be configurable in an expanded configuration and a compressed configuration, and the stent spring can bias the stent to the expanded configuration.

## Claims

1. A stent spring (120) for repairing a pipe comprising:
a substantially tubular mesh structure defining a void (130), the void defining a central axis, the mesh structure comprising one or more strands (140), the one or more strands defining a plurality of openings (142), wherein the stent spring is configurable in an expanded stent spring configuration and a compressed stent spring configuration; and
a tab (960) extending radially inward from the mesh structure into the void, the tab defining a tab opening (962);
an inner stent spring (1122); and
an outer stent spring (1124),
wherein the inner stent spring is connected to the outer stent spring,
wherein the inner stent spring comprises the mesh structure and the tab, and
wherein the outer stent spring is configured to increase a stiffness of the inner stent spring.

2. The stent spring according to claim 1, wherein the inner stent spring and the outer stent spring are substantially similar in shape to the stent spring.

3. The stent spring according to claim 1 or claim 2, wherein the inner stent spring and the outer stent spring are joined together at a plurality of upper bends (1102) and lower bends (1104).

4. The stent spring according to any of claims 1 to 3, further comprising an elastic wire (1512) connected to the one or more strands, the elastic wire configured to increase a flexibility of the stent spring.

5. The stent spring according to claim 4, wherein;
the elastic wire defines a first end and a second end;
the first end of the elastic wire engages a first one of the strands; and
the second end of the elastic wire engages a second one of the strands.

6. The stent spring according to claim 4 or claim 5, wherein:
the first one of the strands defines a first groove;
the second one of the strands defines a second groove;
the first end is received within the first groove; and
the second end is received within the second groove.

7. The stent spring according to claim 5, wherein the elastic wire defines a middle section between the first end and second end, and at least a portion of the middle section is exposed.

8. The stent spring according to any of claims 4 to 7, wherein the elastic wire is positioned on an inner periphery of the stent spring.

9. The stent spring according to any of claims 4 to 8, wherein:
the one or more strands define at least one of an upper bend (1812) and a lower bend (1814); and
the elastic wire is positioned proximate to the at least one of an upper bend and a lower bend.

10. The stent spring according to claim 4, wherein:
a first end of the elastic wire extends through a first groove of the mesh structure and abuts an inner periphery of the stent spring;
a second end of the elastic wire extends through a second groove of the mesh structure and abuts an inner periphery of the stent spring; and
a middle section of the elastic wire is exposed.

11. The stent spring according to any of claims 1 to 10, further comprising a compression mechanism configured to engage the tab to bias the stent spring to the compressed stent spring configuration.

12. A stent (100) for repairing a pipe comprising:
a stent spring (120) for repairing a pipe according to any of claims 1 to 11; and
a seal (170) wrapped around a circumference of the stent spring.

## Patentansprüche

1. Stentfeder (120) zur Reparatur eines Rohrs, umfassend:
eine im Wesentlichen röhrenförmige Netzstruktur, die einen Hohlraum (130) bildet, wobei der Hohlraum eine Mittelachse definiert, wobei die Netzstruktur einen oder mehrere Stränge (140) umfasst, wobei der eine oder die mehreren Stränge eine Vielzahl von Öffnungen (142) bilden, wobei die Stentfeder in einer expandierten Stentfederkonfiguration und einer komprimierten Stentfederkonfiguration konfigurierbar ist; und
eine Lasche (960), die sich von der Netzstruktur radial nach innen in den Hohlraum erstreckt, wobei die Lasche eine Laschenöffnung (962) bildet;
eine innere Stentfeder (1122); und
eine äußere Stentfeder (1124),
wobei die innere Stentfeder mit der äußeren Stentfeder verbunden ist,
wobei die innere Stentfeder die Netzstruktur und die Lasche umfasst, und
wobei die äußere Stentfeder konfiguriert ist, um die Steifigkeit der inneren Stentfeder zu erhöhen.

2. Stentfeder nach Anspruch 1, wobei die innere Stentfeder und die äußere Stentfeder im Wesentlichen die gleiche Form wie die Stentfeder aufweisen.

3. Stentfeder nach Anspruch 1 oder 2, wobei die innere Stentfeder und die äußere Stentfeder an mehreren oberen Biegungen (1102) und unteren Biegungen (1104) miteinander verbunden sind.

4. Stentfeder nach einem der Ansprüche 1 bis 3, ferner umfassend einen elastischen Draht (1512), der mit dem einen oder den mehreren Strängen verbunden ist, wobei der elastische Draht konfiguriert ist, um die Flexibilität der Stentfeder zu erhöhen.

5. Stentfeder nach Anspruch 4, wobei:
der elastische Draht ein erstes Ende und ein zweites Ende definiert;
das erste Ende des elastischen Drahtes in einen ersten der Stränge eingreift; und
das zweite Ende des elastischen Drahtes in einen zweiten der Stränge eingreift.

6. Stentfeder nach Anspruch 4 oder 5, wobei:
der erste der Stränge eine erste Nut definiert;
der zweite der Stränge eine zweite Nut definiert;
das erste Ende in der ersten Nut empfangen wird; und
das zweite Ende in der zweiten Nut empfangen wird.

7. Stentfeder nach Anspruch 5, wobei der elastische Draht einen Mittelabschnitt zwischen dem ersten Ende und dem zweiten Ende definiert und mindestens ein Abschnitt des Mittelabschnitts freiliegt.

8. Stentfeder nach einem der Ansprüche 4 bis 7, wobei der elastische Draht an einem Innenumfang der Stentfeder angeordnet ist.

9. Stentfeder nach einem der Ansprüche 4 bis 8, wobei:
der eine oder die mehreren Stränge mindestens entweder eine obere Biegung (1812) oder eine untere Biegung (1814) definiert; und
der elastische Draht in der Nähe der mindestens einen der oberen Biegung oder oder unteren Biegung angeordnet ist.

10. Stentfeder nach Anspruch 4, wobei:
ein erstes Ende des elastischen Drahtes durch eine erste Nut der Netzstruktur verläuft und an einen inneren Umfang der Stentfeder anstößt;
ein zweites Ende des elastischen Drahtes durch eine zweite Nut der Netzstruktur verläuft und an einen Innenumfang der Stentfeder anstößt; und
ein Mittelabschnitt des elastischen Drahtes freiliegt.

11. Stentfeder nach einem der Ansprüche 1 bis 10, ferner umfassend einen Kompressionsmechanismus, der konfiguriert ist, um in die Lasche einzugreifen, um die Stentfeder in die komprimierte Stentfederkonfiguration vorzuspannen.

12. Stent (100) zur Reparatur eines Rohrs, umfassend:
eine Stentfeder (120) zur Reparatur eines Rohrs nach einem der Ansprüche 1 bis 11; und
eine Dichtung (170), die um den Umfang der Stentfeder gewickelt ist.

## Revendications

1. Ressort de stent (120) permettant de réparer un tuyau comprenant :
une structure maillée essentiellement tubulaire définissant un vide (130), le vide définissant un axe central, la structure maillée comprenant un ou plusieurs brins (140), ledit ou lesdits brins définissant une pluralité d'ouvertures (142), dans lequel le ressort de stent peut être conçu dans une configuration de ressort de stent détendu et une configuration de ressort de stent comprimé ; et
une languette (960) s'étendant radialement vers l'intérieur à partir de la structure maillée dans le vide, la languette définissant une ouverture de languette (962) ;
un ressort de stent interne (1122) ; et
un ressort de stent externe (1124),
dans lequel le ressort de stent interne est relié au ressort de stent externe,
dans lequel le ressort de stent interne comprend la structure maillée et la languette, et
dans lequel le ressort de stent externe est conçu pour augmenter une rigidité du ressort de stent interne.

2. Ressort de stent selon la revendication 1, dans lequel le ressort de stent interne et le ressort de stent externe ont une forme sensiblement similaire à celle du ressort de stent.

3. Ressort de stent selon la revendication 1 ou la revendication 2, dans lequel le ressort de stent interne et le ressort de stent externe sont assemblés au niveau d'une pluralité de courbures supérieures (1102) et de courbures inférieures (1104).

4. Ressort de stent selon l'une quelconque des revendications 1 à 3, comprenant en outre un fil élastique (1512) relié audit ou auxdits brins, le fil élastique étant conçu pour augmenter la souplesse du ressort de stent.

5. Ressort de stent selon la revendication 4, dans lequel ;
le fil élastique définit une première extrémité et une seconde extrémité ;
la première extrémité du fil élastique se met en prise avec un premier des brins ; et
la seconde extrémité du fil élastique se met en prise avec un second des brins.

6. Ressort de stent selon la revendication 4 ou la revendication 5, dans lequel :
le premier des brins définit une première rainure ;
le second des brins définit une seconde rainure ;
la première extrémité est reçue à l'intérieur de la première rainure ; et
la seconde extrémité est reçue à l'intérieur de la seconde rainure.

7. Ressort de stent selon la revendication 5, dans lequel le fil élastique définit une section centrale entre la première extrémité et la seconde extrémité, et au moins une partie de la section centrale est exposée.

8. Ressort de stent selon l'une quelconque des revendications 4 à 7, dans lequel le fil élastique est positionné sur une périphérie interne du ressort de stent.

9. Ressort de stent selon l'une quelconque des revendications 4 à 8, dans lequel :
ledit ou lesdits brins définissent au moins l'une parmi une courbure supérieure (1812) et une courbure inférieure (1814) ; et
le fil élastique est positionné à proximité de l'au moins l'une parmi une courbure supérieure et une courbure inférieure.

10. Ressort de stent selon la revendication 4, dans lequel :
une première extrémité du fil élastique s'étend à travers une première rainure de la structure maillée et vient en butée contre la périphérie interne du ressort de stent ;
une seconde extrémité du fil élastique s'étend à travers une seconde rainure de la structure maillée et vient en butée contre la périphérie interne du ressort de stent ; et
une partie centrale du fil élastique est exposée.

11. Ressort de stent selon l'une quelconque des revendications 1 à 10, comprenant en outre un mécanisme de compression conçu pour se mettre en prise avec la languette afin de solliciter le ressort de stent vers la configuration de ressort de stent comprimé.

12. Stent (100) permettant de réparer un tuyau comprenant :
un ressort de stent (120) permettant de réparer un tuyau selon l'une quelconque des revendications 1 à 11 ; et
un joint d'étanchéité (170) enroulé autour de la circonférence du ressort de stent.
